# FASCICULE DE BREVET EUROPEEN

(11) **EP 3 347 255 B1**
(45) Date de publication et mention de la délivrance du brevet: **19.06.2019**
(21) Numéro de dépôt: 16770061.6
(22) Date de dépôt: 30.08.2016
(51) Int. Cl.: B60W 50/12, A61B 5/18, A61B 5/00, B60K 28/06, B60W 40/08, B60W 50/00, H04L 29/08, A61B 5/0476, G16H 50/20, G16H 40/63, G16H 40/67

(54) **PROCÉDÉ ET DISPOSITIF D'AIDE À LA CONDUITE UTILISANT UN PÉRIPHÉRIQUE DE MESURE D'AU MOINS UN PARAMÈTRE PHYSIOLOGIQUE**
VERFAHREN UND VORRICHTUNG ZUR FAHRERUNTERSTÜTZUNG UNTER VERWENDUNG EINES PERIPHERIEGERÄTS ZUR MESSUNG VON MINDESTENS EINEM PHYSIOLOGISCHEN PARAMETER
METHOD AND DEVICE FOR DRIVING ASSISTANCE USING A PERIPHERAL FOR MEASURING AT LEAST ONE PHYSIOLOGICAL PARAMETER

(30) Priorité: 09.09.2015 FR 1558369
(43) Date de publication de la demande: 18.07.2018
(73) Titulaire: PSA AUTOMOBILES S.A., 78300 Poissy (FR)
(72) Inventeur: MOREL-GARDIEN, Virginie, 92400 Courbevoie (FR); BOUZAT, Lise, 78480 Verneuil sur Seine (FR)
(74) Mandataire: Jeannin, Laurent Jean-Pierre
(86) Numéro de dépôt international: PCT/FR2016/052147
(87) Numéro de publication internationale: WO 2017/042452

(56) Documents cités:
- DE-A1-102011 089 938
- DE-A1-102011 109 564
- DE-A1-102012 205 808
- DE-A1-102012 220 655
- US-A1- 2006 011 399
- US-A1- 2013 226 408
- US-A1- 2014 240 132

## Description

L'invention concerne l'assistance aux conducteurs et plus particulièrement aux conducteurs susceptibles de connaître un problème de santé permanent ou momentané.

On connaît par le document DE102012002037 un dispositif comportant des capteurs distribués sur un dossier de siège de véhicule pour mesurer des signes vitaux d'un conducteur. En cas de défaillance du conducteur, le dispositif est capable d'émettre une alerte.

Un tel dispositif a notamment pour inconvénient de ne pas détecter certaines pathologies. De plus, le dispositif n'est pas personnalisable par l'utilisateur. Il ne permet pas de mettre à disposition du conducteur toutes les ressources du véhicule dont celui-ci pourrait avoir besoin alors que son état de santé est dégradé.

On connait aussi par le document DE102011089938 une méthode consistant à traiter les spécifications d'un véhicule à partir d'informations destinées à l'opérateur d'un véhicule et/ou d'informations de contrôle produites à partir d'une sortie de véhicule. L'efficacité individuelle de l'opérateur du véhicule est traitée pour reproduire des données d'entrée supplémentaires, c'est-à-dire les paramètres physiologiques de l'opérateur du véhicule.

On connait aussi par le document US20060011399 un système et une méthode pour contrôler le fonctionnement du véhicule en fonction des expressions faciales et de l'état physique du conducteur.

L'invention a donc pour but de remédier aux problèmes précités. Elle propose plus précisément à cet effet un procédé d'assistance à la conduite d'un véhicule, mis en oeuvre par un dispositif mobile et comportant des étapes de :
- Connexion avec un périphérique de mesure d'au moins un paramètre physiologique d'un conducteur,
- Réception des mesures du paramètre physiologique,
- Détermination d'un état de santé dudit conducteur à partir des paramètres physiologiques reçus,

Caractérisé en ce qu'il comporte en outre des étapes de :
- Connexion avec un calculateur d'un véhicule,
- Sélection d'un profil associé audit conducteur, ledit profil comportant des associations entre des états de santé prédéfinis, d'une part, et des paramètres de configuration du véhicule d'autre part,
- Détermination de paramètres de configuration du véhicule à partir de l'état de santé déterminé et du profil sélectionné,
- Transmission au calculateur du véhicule des paramètres de configuration déterminés.

Le dispositif selon l'invention permet d'établir une interface entre l'état de santé du conducteur et le véhicule. Le véhicule s'adapte à l'état de santé du conducteur en fonction des paramètres de configuration qui sont renseignés dans le profil.

Le dispositif selon l'invention a aussi pour avantage de fonctionner de façon autonome sans nécessiter une connexion à Internet.

En outre, aucune des données médicales n'est transmise vers le véhicule. En effet, seul les paramètres de configuration sont transmis vers le véhicule. De la sorte la confidentialité des données est assurée.

Selon un mode de réalisation de l'invention, l'étape de réception des mesures du paramètre physiologique comporte un enregistrement d'électroencéphalographie, le paramètre physiologique mesuré étant représentatif de l'activité électrique du cerveau du conducteur. Cette caractéristique a pour avantage de permettre de détecter une dégradation d'un état de santé uniquement décelable via ce paramètre physiologique.

Selon une caractéristique de l'invention, l'un des états de santé prédéfinis correspond à un risque de crise d'épilepsie. Cette caractéristique permet d'adapter la configuration du véhicule dans le cas spécifique d'un risque de crise d'épilepsie.

Selon une caractéristique de l'invention, l'un des états de santé prédéfinis correspond à un risque de crise d'hypoglycémie ou d'hyperglycémie de conducteurs diabétiques. Cette caractéristique permet d'adapter la configuration du véhicule dans le cas spécifique d'un risque de crise d'hypoglycémie ou d'hyperglycémie.

Selon une caractéristique de l'invention, le périphérique de mesure d'au moins un paramètre physiologique est un vêtement connecté. Cette caractéristique permet de placer des capteurs au plus près du corps et d'obtenir des mesures de paramètres impossibles autrement.

Selon un autre mode de réalisation de l'invention, l'étape de réception des mesures du paramètre physiologique comporte l'enregistrement de pulsations cardiaques du conducteur.

Selon une caractéristique de l'invention, le périphérique de mesure d'au moins un paramètre physiologique est un bracelet ou une montre connecté.

Selon une caractéristique de l'invention, le périphérique de mesure d'au moins un paramètre physiologique est un capteur externe permettant la l'enregistrement, la mesure et l'analyse vocale de la parole du conducteur.

Selon une caractéristique de l'invention, le périphérique de mesure d'au moins un paramètre physiologique est un capteur externe de type caméra permettant la mesure et l'analyse visuelle comportementale du conducteur.

Selon une autre caractéristique de l'invention, le profil comporte au moins trois états de santé prédéfinis différents : un premier état de santé correspondant à un état satisfaisant du conducteur, un deuxième état de santé nécessitant une surveillance et un troisième état de santé dans lequel le conducteur n'est plus en mesure de conduire.

Cette caractéristique permet de considérer de façon graduelle la santé du conducteur et permet d'adapter en conséquence les paramètres de configuration du véhicule.

Selon une autre caractéristique de l'invention, le profil est en outre associé audit véhicule.

L'utilisation d'un profil spécifique pour chaque véhicule permet d'adapter les paramètres de configuration en fonction des équipements disponibles dans le véhicule (présence ou non d'un appel d'urgence ou d'un mode de conduite autonome).

L'invention concerne aussi un dispositif d'assistance à la conduite d'un véhicule, comportant :
- des moyens de connexion avec un périphérique de mesure d'au moins un paramètre physiologique d'un conducteur,
- des moyens de réception des mesures du paramètre physiologique,
- des moyens de détermination d'un état de santé dudit conducteur à partir des paramètres physiologiques reçus,

Caractérisé en ce qu'il comporte en outre :
- des moyens de connexion avec un calculateur d'un véhicule,
- des moyens de sélection d'un profil associé audit conducteur, ledit profil comportant des associations entre des états de santé prédéfinis, d'une part, et des paramètres de configuration du véhicule d'autre part,
- des moyens de détermination des paramètres de configuration du véhicule à partir de l'état de santé déterminé,
- des moyens de transmission au calculateur des paramètres de configuration déterminé.

L'invention concerne aussi un système d'aide à la conduite d'un véhicule comportant un calculateur caractérisé en ce qu'il comporte en outre un périphérique de mesure d'au moins un paramètre physiologique d'un conducteur et un dispositif mobile selon l'invention, connecté, d'une part, audit périphérique de mesure et, d'autre part, audit calculateur.

D'autres caractéristiques et avantages de l'invention apparaîtront à l'examen de la description détaillée ci-après, et des dessins annexés, sur lesquels:
- la figure 1 illustre une vue schématique d'un système d'aide à la conduite selon l'invention ;

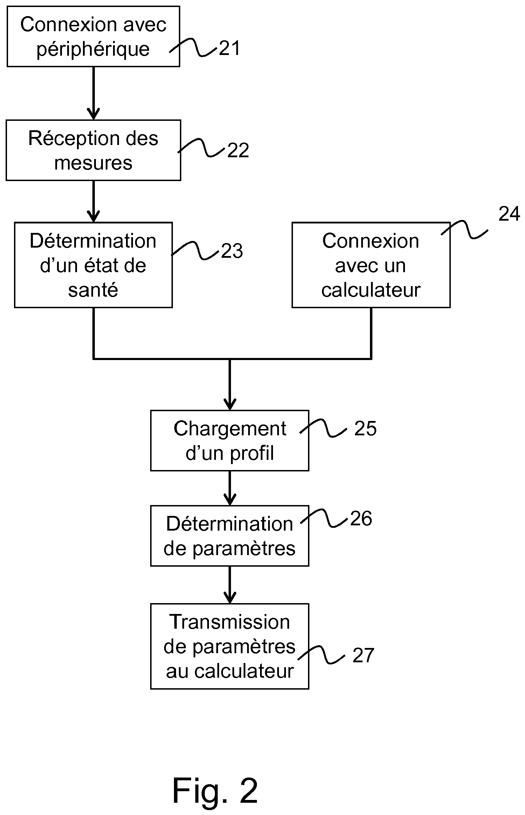

- la figure 2 montre un logigramme illustrant le procédé selon l'invention.

Les dessins annexés pourront non seulement servir à compléter l'invention, mais aussi contribuer à sa définition, le cas échéant.

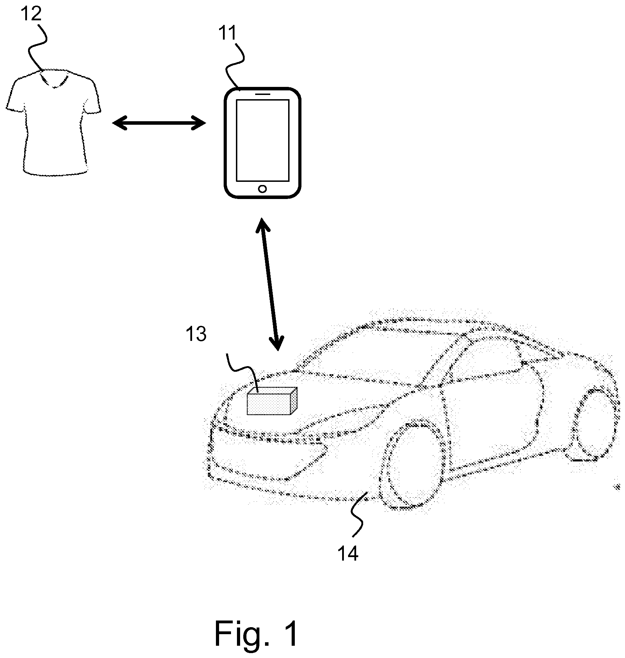

En référence à la figure 1, le système 10 d'aide à la conduite selon l'invention comporte un dispositif mobile 11, un périphérique 12 de mesure d'au moins un paramètre physiologique d'un conducteur et un calculateur 13 embarqué à bord d'un véhicule 14.

Dans ce qui suit, on considère à titre d'exemple non limitatif que le véhicule 14 est de type automobile. Mais l'invention n'est pas limitée à ce mode de réalisation. En effet, elle concerne tout type de véhicule, i.e thermique, électrique, les 2 roues de types : scooters, cycles avec ou sans assistance électrique.

Le calculateur 13 embarqué à bord du véhicule comporte au moins un processeur et au moins une mémoire. Le calculateur 13 comprend en outre des moyens de communications sans fil ou/et filaire. Le dispositif mobile 11 et le calculateur 13 échangent des données par le biais d'une communication sans fil (par exemple Bluetooth ou wifi) ou par une communication filaire.

Le calculateur 13 comporte des moyens pour recevoir des paramètres de configuration et des moyens pour commander l'application de ces paramètres de configuration aux différents équipements du véhicule.

Le calculateur 13 est par exemple apte à commander : l'activation ou la désactivation de la climatisation de l'habitacle, l'allumage ou l'extinction d'un éclairage de l'habitacle, l'envoi d'un appel d'urgence, l'activation d'un mode de conduite autonome prenant en charge partiellement ou en totalité les commandes du véhicule.

Le périphérique 12 est apte à mesurer et / ou analyser un ou plusieurs paramètres physiologiques d'un conducteur comme par exemple : la fréquence cardiaque, l'activité électrique cérébrale, la fréquence de la respiration ou la glycémie. Le périphérique 12 comporte en outre des moyens de connexion au dispositif mobile 11. Le dispositif mobile 11 et le périphérique 12 échangent des données par le biais d'une communication sans fil (par exemple Bluetooth ou wifi) ou par une communication filaire.

Selon un mode de réalisation, le périphérique 12 est un vêtement connecté apte à mesurer un paramètre physiologique représentatif de l'activité électrique cérébrale d'un conducteur et permettant d'établir un électro-encéphalogramme.

On connaît, par exemple, par le document « BIOSERENITY RAISES 3 MILLION EUROS IN SEED ROUND », (publié le 04/06/2015 et disponible sur le site internet http://www.bioserenity.com/img/press_en_2015-06-04_Bioserenity.pdf) un vêtement connecté associé à une application mobile permettant l'enregistrement d'électro-encéphalographie en continue. Le vêtement est équipé de capteurs biométriques intégrés enregistrant les paramètres corporels. Il comprend un T-shirt et un bonnet. L'application mobile analyse les données venant du vêtement.

Selon un autre mode de réalisation, le périphérique 12 est un bracelet connecté apte à mesurer la fréquence cardiaque.

Selon un autre mode de réalisation, le périphérique 12 est une montre connectée apte à mesurer la fréquence cardiaque.

Selon un autre mode de réalisation, le périphérique 12 est une caméra embarquée pour mesurer la fréquence cardiaque, le niveau d'éveil du conducteur.

Selon un autre mode de réalisation, le périphérique 12 est un micro embarqué apte à enregistrer la voix du conducteur pour réaliser l'analyse vocale du conducteur concernant son état d'éveil voire son état de santé (ex : détection d'AVC et / ou de malaise cardiaque).

Le dispositif mobile 11 est par exemple un téléphone mobile intelligent (ou « smartphone »), une tablette tactile ou un ordinateur portable comportant :
- des moyens de connexion avec un périphérique 12 de mesure d'au moins un paramètre physiologique d'un conducteur,
- des moyens de réception des mesures du paramètre physiologique,
- des moyens de détermination d'un état de santé dudit conducteur à partir des paramètres physiologiques reçus.

Le dispositif mobile 11 comporte en outre :
- des moyens de connexion avec un calculateur 13 d'un véhicule 14,
- des moyens de sélection d'un profil associé audit conducteur, ledit profil comportant des associations entre des états de santé prédéfinis, d'une part, et des paramètres de configuration du véhicule 14 d'autre part,
- des moyens de détermination des paramètres de configuration du véhicule 14 à partir de l'état de santé déterminé,
- des moyens de transmission au calculateur 13 des paramètres de configuration déterminés.

L'invention concerne aussi un procédé d'assistance à la conduite d'un véhicule 14. Le procédé selon l'invention est mis en oeuvre par le dispositif mobile 11 et comporte les étapes suivantes.

Le procédé selon l'invention comporte tout d'abord une étape de connexion 21 du dispositif mobile 11 avec le périphérique 12 de mesure d'au moins un paramètre physiologique du conducteur. La connexion entre ces deux équipements 11, 12 est par exemple une connexion sans fils de type Bluetooth.

Ensuite, le dispositif mobile 11 réceptionne 22 des mesures du paramètre physiologique provenant du périphérique de mesure.

Le dispositif mobile est alors en mesure de déterminer 23 un état de santé du conducteur à partir des paramètres physiologiques reçus.

Selon un mode de réalisation avantageux, la réception 22 des mesures du paramètre physiologique comporte un enregistrement d'électroencéphalographie. Le paramètre physiologique mesuré est alors représentatif de l'activité électrique du cerveau du conducteur. Dans ce cas, un des états de santé prédéfinis peut correspondre à un risque de crise d'épilepsie. Autrement dit, le dispositif mobile est en mesure d'identifier une crise d'épilepsie imminente.

L'utilisation d'un vêtement connecté comportant un élément à disposer sur la tête du conducteur (par exemple un bonnet) est particulièrement adaptée à ce mode de réalisation.

De façon alternative ou de façon complémentaire au mode de réalisation précédant, la réception 22 des mesures du paramètre physiologique comporte l'enregistrement de pulsation cardiaque du conducteur. Dans ce cas, un des états de santé prédéfinis correspond par exemple à des palpitations cardiaques.

La mesure des pulsations cardiaques du conducteur peut être effectuée par un bracelet connecté, par le vêtement connecté précédemment évoqué ou par tout autre moyen de mesure adapté.

Selon un mode de réalisation de l'invention, le profil comporte au moins trois états de santé prédéfinis différents :
- un premier état de santé correspondant à un état satisfaisant du conducteur,
- un deuxième état de santé nécessitant une surveillance et
- un troisième état de santé pour dans lequel le conducteur n'est plus en mesure de conduire.

Dans ce mode de réalisation, le dispositif mobile signale par exemple l'état de santé par un affichage d'un code couleur : vert pour le premier état de santé, orange pour le deuxième et rouge pour le troisième.

Le procédé selon l'invention comporte aussi une étape de connexion 24 du dispositif mobile 11 avec le calculateur 13 du véhicule 14. La connexion entre ces deux équipements 11, 13 est par exemple une connexion sans fils de type Bluetooth ou Wifi.

Le dispositif mobile sélectionne alors un profil associé, d'une part, au conducteur et, d'autre part, au véhicule 14. Le profil comporte des associations entre des états de santé prédéfinis, d'une part, et des paramètres de configuration du véhicule 14 d'autre part.

Ce profil permet d'établir une relation entre un état de santé détecté par le dispositif mobile 11 et une action ou une configuration du véhicule 14 en réponse à cet état de santé. Par exemple, le profil comporte une association entre l'état de santé « risque crise d'épilepsie » avec les paramètres de configuration du véhicule 14 permettant l'émission d'un appel d'urgence par le véhicule et/ou l'activation d'un mode de conduite autonome du véhicule 14.

Le profil comporte par exemple les associations suivantes :
- Pour le premier état de santé, le conducteur va bien, aucun paramètre du véhicule n'est modifié ;
- Pour le deuxième état de santé, les paramètres de configuration modifiés sont : la température de l'habitacle (consigne vers une température prédéfinie), sélection d'un programme musical ou audio , sélection d'une position de conduite prédéfinie, sélection d'une ambiance lumineuse dans l'habitacle, démarrage de la ventilation ;
- Pour le troisième état de santé, les paramètres de configuration modifiés sont : appel d'urgence, parking automatique sur le bas-côté de la route, appel téléphonique à une liste de contact pré-renseignée.

Ainsi lorsqu'un état de santé est identifié par le dispositif mobile 11, celui-ci détermine 26 les paramètres de configuration du véhicule 14 en consultant le profil sélectionné.

Le dispositif mobile 11 transmet 27 au calculateur 13 du véhicule 14 des paramètres de configuration déterminés.

Dans l'exemple, le dispositif mobile 11 transmet 27 au calculateur 13 des paramètres de configuration correspondant à l'émission d'un appel d'urgence. En réponse, le calculateur 13 commande l'émission d'un appel d'urgence.

## Revendications

1. Procédé d'assistance à la conduite d'un véhicule (14), mis en oeuvre par un dispositif mobile (11) et comportant des étapes de :
- Connexion (21) avec un périphérique (12) de mesure d'au moins un paramètre physiologique d'un conducteur,
- Réception (22) des mesures du paramètre physiologique,
- Détermination (23) d'un état de santé dudit conducteur à partir des paramètres physiologiques reçus,
- Connexion (24) avec un calculateur (13) d'un véhicule (14),
- Sélection (25) d'un profil associé audit conducteur,
- Détermination (26) de paramètres de configuration du véhicule (14) à partir de l'état de santé déterminé et du profil sélectionné,
- Transmission (27) au calculateur (13) du véhicule (14) des paramètres de configuration déterminés, **caractérisé en ce que**
le profil comporte des associations entre des états de santé prédéfinis, d'une part, et des paramètres de configuration du véhicule (14) d'autre part.

2. Procédé d'assistance à la conduite selon la revendication 1, **caractérisé en ce que** l'étape de réception (22) des mesures du paramètre physiologique comporte un enregistrement d'électro-encéphalographie, le paramètre physiologique mesuré étant représentatif de l'activité électrique du cerveau du conducteur.

3. Procédé d'assistance à la conduite selon la revendication 2, **caractérisé en ce qu'**un des états de santé prédéfinis correspond à un risque de crise d'épilepsie.

4. Procédé d'assistance à la conduite selon l'une des revendications précédentes, **caractérisé en ce que** le périphérique (12) de mesure d'au moins un paramètre physiologique est un vêtement connecté.

5. Procédé d'assistance à la conduite selon la revendication 1, **caractérisé en ce que** l'étape de réception (22) des mesures du paramètre physiologique comporte l'enregistrement de pulsations cardiaques du conducteur.

6. Procédé d'assistance à la conduite selon la revendication 5, **caractérisé en ce que** le périphérique (12) de mesure d'au moins un paramètre physiologique est un bracelet connecté ou une montre connectée.

7. Procédé d'assistance à la conduite selon l'une des revendications précédentes, **caractérisé en ce que** le profil comporte au moins trois états de santé prédéfinis différents : un premier état de santé correspondant à un état satisfaisant du conducteur, un deuxième état de santé nécessitant une surveillance et un troisième état de santé pour dans lequel le conducteur n'est plus en mesure de conduire.

8. Procédé d'assistance à la conduite selon l'une des revendications précédentes, **caractérisé en ce que** le profil est en outre associé audit véhicule (14).

9. Dispositif (11) d'assistance à la conduite d'un véhicule (14), comportant :
- des moyens de connexion avec un périphérique (12) de mesure d'au moins un paramètre physiologique d'un conducteur,
- des moyens de réception des mesures du paramètre physiologique,
- des moyens de détermination d'un état de santé dudit conducteur à partir des paramètres physiologiques reçus,
- des moyens de connexion avec un calculateur (13) d'un véhicule (14),
- des moyens de sélection d'un profil associé audit conducteur,
- des moyens de détermination des paramètres de configuration du véhicule à partir de l'état de santé déterminé,
- des moyens de transmission au calculateur (13) des paramètres de configuration déterminé, **caractérisé en ce que**
le profil comporte des associations entre des états de santé prédéfinis, d'une part, et des paramètres de configuration du véhicule (14) d'autre part.

10. Système (10) d'aide à la conduite d'un véhicule (14) comportant un calculateur (13) **caractérisé en ce qu'**il comporte en outre un périphérique (12) de mesure d'au moins un paramètre physiologique d'un conducteur et un dispositif mobile (11) selon la revendication précédente, connecté d'une part audit périphérique (12) de mesure et d'autre part audit calculateur (13).

## Patentansprüche

1. Fahrunterstützungsverfahren eines Fahrzeugs (14), das von einer mobilen Vorrichtung (11) umgesetzt wird und folgende Schritte umfasst:
- Verbinden (21) mit einem Peripheriegerät (12) zur Messung mindestens eines physiologischen Parameters eines Fahrers,
- Empfang (22) von Messungen des physiologischen Parameters,
- Bestimmen (23) eines Gesundheitszustands des Fahrers ausgehend von den empfangenen physiologischen Parametern,
- Verbindung (24) mit einem Rechner (13) eines Fahrzeugs (14),
- Auswahl (25) eines Profils, das mit dem Fahrer assoziiert ist,
- Bestimmen (26) von Konfigurationsparametern des Fahrzeugs (14) ausgehend von dem bestimmten Gesundheitszustand und dem ausgewählten Profil,
- Übertragen (27) an den Rechner (13) des Fahrzeugs (14) der bestimmten Konfigurationsparameter,
**dadurch gekennzeichnet, dass**
es Assoziationen zwischen vordefinierten Gesundheitszuständen einerseits und Konfigurationsparametern des Fahrzeugs (14) andererseits umfasst.

2. Fahrunterstützungsverfahren nach Anspruch 1, **dadurch gekennzeichnet, dass** der Empfangsschritt (22) der Messungen des physiologischen Parameters eine Elektroenzephalografieaufzeichnung umfasst, wobei der gemessene physiologische Parameter für die elektrische Aktivität des Gehirns des Fahrers repräsentativ ist.

3. Fahrunterstützungsverfahren nach Anspruch 2, **dadurch gekennzeichnet, dass** einer der vordefinierten Gesundheitszustände einer Epilepsiekrisengefahr entspricht.

4. Fahrunterstützungsverfahren nach einem der vorstehenden Ansprüche, **dadurch gekennzeichnet, dass** das Peripheriegerät (12) zur Messung mindestens eines physiologischen Parameters ein vernetztes Kleidungsstück ist.

5. Fahrunterstützungsverfahren nach Anspruch 1, **dadurch gekennzeichnet, dass** der Empfangsschritt (22) der Messungen des physiologischen Parameters das Aufzeichnen von Herzschlägen des Fahrers ist.

6. Fahrunterstützungsverfahren nach Anspruch 5, **dadurch gekennzeichnet, dass** das Peripheriegerät (12) zur Messung mindestens eines physiologischen Parameters ein vernetztes Armband oder eine vernetzte Uhr ist.

7. Fahrunterstützungsverfahren nach einem der vorstehenden Ansprüche, **dadurch gekennzeichnet, dass** das Profil mindestens drei vorbestimmte Gesundheitszustände umfasst: einen ersten Gesundheitszustand, der einem zufriedenstellenden Zustand des Fahrers entspricht, einen zweiten Gesundheitszustand, der eine Überwachung erfordert, und einen dritten Gesundheitszustand, bei dem der Fahrer nicht mehr in der Lage ist, zu lenken.

8. Fahrunterstützungsverfahren nach einem der vorstehenden Ansprüche, **dadurch gekennzeichnet, dass** das Profil außerdem mit dem Fahrzeug (14) assoziiert ist.

9. Fahrunterstützungsvorrichtung (11) eines Fahrzeugs (14), die Folgendes umfasst:
- Mittel zum Verbinden mit einem Peripheriegerät (12) zur Messung mindestens eines physiologischen Parameters eines Fahrers,
- Mittel zum Empfangen der Messungen des physiologischen Parameters,
- Mittel zum Bestimmen eines Gesundheitszustands des Fahrers ausgehend von den empfangenen physiologischen Parametern,
- Mittel zum Verbinden mit einem Rechner (13) eines Fahrzeugs (14),
- Mittel zum Auswählen eines Profils, das mit dem Fahrer assoziiert ist,
- Mittel zum Bestimmen der Konfigurationsparameter des Fahrzeugs ausgehend von dem bestimmten Gesundheitszustand,
- Mittel zum Übertragen der bestimmten Konfigurationsparameter zu dem Rechner (13),
**dadurch gekennzeichnet, dass**
das Profil Assoziationen zwischen vordefinierten Gesundheitszuständen einerseits und Konfigurationsparametern des Fahrzeugs (14) andererseits umfasst.

10. Fahrunterstützungssystem (10) eines Fahrzeugs (14), das einen Rechner (13) umfasst, **dadurch gekennzeichnet, dass** es außerdem ein Peripheriegerät (12) zur Messung mindestens eines physiologischen Parameters eines Fahrers sowie eine mobile Vorrichtung (11) gemäß dem vorstehenden Anspruch, die einerseits an das Peripheriegerät (12) zur Messung und andererseits an den Rechner (13) angeschlossen ist, umfasst.

## Claims

1. A method to assist with the driving of a vehicle (14), implemented by a mobile device (11) and comprising steps of:
- connecting (21) with a peripheral (12) for measuring at least one physiological parameter of a driver,
- receiving (22) measurements of the physiological parameter,
- determining (23) a state of health of said driver from the physiological parameters received,
- connecting (24) with a computer (13) of a vehicle (14),
- selecting (25) a profile associated with said driver,
- determining (26) configuration parameters of the vehicle (14) from the determined state of health and the selected profile,
- transmitting (27) the determined configuration parameters to the computer (13) of the vehicle (14),
**characterized in that**
the profile comprises associations between predefined states of health on the one hand, and configuration parameters of the vehicle (14) on the other hand.

2. The method to assist with driving according to claim 1, **characterized in that** the step of receiving (22) measurements of the physiological parameter comprises an electro-encephalography recording, the measured physiological parameter being representative of the electrical activity of the driver's brain.

3. The method to assist with driving according to claim 2, **characterized in that** one of the predefined states of health corresponds to a risk of an epileptic seizure.

4. The method to assist with driving according to one of the preceding claims, **characterized in that** the peripheral (12) for measuring at least one physiological parameter is a connected garment.

5. The method to assist with driving according to claim 1, **characterized in that** the step of receiving (22) measurements of the physiological parameter comprises the recording of the driver's heartbeats.

6. The method to assist with driving according to claim 5, **characterized in that** the peripheral (12) for measuring at least one physiological parameter is a connected bracelet or a connected watch.

7. The method to assist with driving according to one of the preceding claims, **characterized in that** the profile comprises at least three different predefined states of health: a first state of health corresponding to a satisfactory state of the driver, a second state of health necessitating a monitoring, and a third state of health in which the driver is no longer able to drive.

8. The method to assist with driving according to one of the preceding claims, **characterized in that** the profile is, in addition, associated with said vehicle (14) .

9. A device (11) to assist with the driving of a vehicle (14), comprising:
- means for connection with a peripheral (12) for measuring at least one physiological parameter of a driver,
- means for receiving measurements of the physiological parameter,
- means for determining a state of health of said driver from the received physiological parameters,
- means for connection with a computer (13) of a vehicle (14),
- means for selecting a profile associated with said driver,
- means for determining configuration parameters of the vehicle from the determined state of health,
- means for transmission to the computer (13) of the determined configuration parameters,
**characterized in that**
the profile comprises associations between predefined states of health, on the one hand, and configuration parameters of the vehicle (14) on the other hand.

10. A system (10) for driving assistance of a vehicle (14) comprising a computer (13), **characterized in that** it comprises in addition a peripheral (12) for measuring at least one physiological parameter of a driver and a mobile device (11) according to the preceding claim, connected on the one hand to said peripheral (12) for measuring, and on the other hand to said computer (13) .
